# EUROPEAN PATENT APPLICATION

(11) **EP 1 571 195 A1**
(43) Date of publication of application: **07.09.2005**
(21) Application number: 03774145.1
(22) Date of filing: 21.11.2003
(51) Int. Cl.: C09K 15/06, C11B 5/00, A61K 31/202, A61K 31/232, A61P 37/08, A61K 31/341

(54) **EXTERNAL COMPOSITION CONTAINING HIGHLY UNSATURATED FATTY ACID OR ITS SALT OR ESTER**

(30) Priority: 22.11.2002 JP 2002339906
(71) Applicant: NIPPON SUISAN KAISHA, LTD., Tokyo 100-0004 (JP)
(72) Inventor: DOISAKI, Nobushige, c/o Nippon Suisan Kaisha, Ltd., Hachioji-shi, Tokyo 192-0906 (JP); YAMAGUCHI, Hideaki, c/o Nippon Suisan Kaisha, Ltd., Hachioji-shi, Tokyo 192-0906 (JP); JINNO, Shuji, c/o Nippon Suisan Kaisha, Ltd., Hachioji-shi, Tokyo 192-0906 (JP); MUKAE, Katsuya, Fukuoka-shi, Fukuoka 811-0211 (JP)
(74) Representative: Vossius & Partner
(86) International application number: PCT/JP2003/014905
(87) International publication number: WO 2004/048497

(57) **Abstract**

To a polyunsaturated fatty acid or its salt or ester was added an antioxidative sesame component and ascorbic acid or an ascorbyl fatty acid ester. To the polyunsaturated fatty acid or its salt or ester, or an oil or fat containing the polyunsaturated fatty acid or its salt or ester having an peroxide value (PV) of 3.0 meq/kg or less and an acid value (AV) of 1.0 or less, and no odor in terms of sensory testing was mixed with an antioxidative sesame component, ascorbic acid or an ascorbyl fatty acid ester, and an external base material, under conditions preventing oxygen from mixing, and the mixture is quickly placed in a hermetic container.

The above method provides an external composition containing a polyunsaturated fatty acid or its salt or ester, exhibiting enhanced oxidative stability, and consequently preventing the generation of peculiar odor and peroxides resulting from the oxidation of the polyunsaturated fatty acid.

## Description

### TECHNICAL FIELD

The present invention relates to an external composition containing a polyunsaturated fatty acid or its salt or ester, exhibiting enhanced oxidative stability, and consequently preventing the generation of peculiar odor and peroxides resulting from the oxidation of the polyunsaturated fatty acid. Specifically, the present invention relates to an external composition exhibiting enhanced oxidative stability, prepared by adding an antioxidative component of sesame and an ascorbic acid or ascorbyl fatty acid ester to the polyunsaturated fatty acid or its salt or ester, and which is not affected by oxidation. For example, it does not generate an odor resulting from oxidation when it is applied to skin. The present invention also relates to an external composition used for curing inflammatory skin diseases.

In the present invention, the polyunsaturated fatty acid refers to a fatty acid having at least three double bonds.

In the present invention, the external composition refers to a composition applied to skin, such as an external drug, an external quasi drug, or a cosmetic preparation.

The "external" means being intended to be applied to skin.

The "drug" is intended for use for diagnosis, cure, or prevention of human and animal diseases. The "cosmetic preparation" is intended to be applied or sprayed to skin so as to clean or spruce the human body, increase charm, change physical appearance, or maintain healthy skin or hair, and its effect is moderate for human. The "quasi drug" stands midway between the drug and the cosmetic preparation, and exhibits clearer efficacy than the cosmetic preparation and prevents side effects and skin trouble. The composition of the present invention covers these categories, or their equivalents if such categories are not defined depending on the country.

### BACKGROUND ART

Allergodermia has recently been increasing rapidly in association with change and complication in living environment and change in dietary habits. Exemplary allergodermias include atopic dermatitis, psoriasis, and contact dermatitis. Various medical treatments have been conventionally made for these types of allergodermia. For example, allergens are avoided by dietary cure and improvement of living environments, and antiallergic drugs and steroid are administered. However, no decisive cure has not yet been found for allergodermia, and accordingly a safe therapeutic drug is desired which produces satisfactory therapeutic effects without side effects.

It has been found that eicosapentaenoic acid and docosahexaenoic acid, which are polyunsaturated fatty acids mainly contained in fish oil, have various effects, such as of preventing high blood pressure and skin aging, and they have been used in medical drugs and food with health-promoting benefits. These fatty acids are expected to produce a therapeutic effect for allergic disease. In fact, it has been reported that atopic dermatitis had been remedied by internally administering eicosapentaenoic acid or applying it to skin (Brit Respir J. 1987, 117,463-469; The Journal of Medical Investigation 1999, 46, 173-177).

It is considered that the antiinflammatory action and immunosuppressive action of eicosapentaenoic acid (EPA) are effective for allergic diseases. It is known that fatty acid compositions on the membranes of inflammatory cells and immunocytes are highly affected by diet. It has been considered that, in particular, constituents of the polyunsaturated fatty acid are associated with inflammation and immunity, because they are changed depending on fatty acids derived from diet. Arachidonic acid (AA) is a precursor of prostaglandin and leukotriene, consequently playing a significant role to control inflammation and immunity. Fish oil contains EPA. The ingestion of fish oil causes AA on cell membranes to be replaced with EPA, consequently reducing the production of mediators from the AA. Also, it is known that EPA serves as a substrate of cyclooxygenase and lipoxygenase, and that metabolites derived from EPA have physiological effects different from metabolites derived from AA. One of the most expected effects of EPA on various allergic diseases is such contradictory action to the mediators derived from AA. In addition, it has been reported that EPA exhibits various inhibiting effects on immunocytes. Since EPA probably has a moderate immunomodulatory effect, but not strong effect, and hardly produces side effects, it can be administered for a long term. Thus, EPA is expected to be used as a basic therapeutic drug for chronic immunological diseases.

Since polyunsaturated fatty acids, especially eicosapentaenoic acid (EPA) and docosahexaenoic acid (DHA), have various types of bioactivity, they are widely used in medical drugs, food, and others. However, oils and fats containing polyunsaturated fatty acids exhibit very low oxidative stability. Accordingly, they are encapsulated for oral administration as medical drugs, or used for food in combination with various types of antioxidant or masking agent. However, no perfect method has been found yet, and thus there are various limits in use, such as of product type, distribution temperature, content, and storage conditions. A drug product is desired which can be used as an external preparation having sufficient oxidative stability.

Although many patent applications have been filed for use of EPA or the like as an antiallergic drug, no invention has been put in practical use (for example, Japanese Unexamined Patent Application Publication Nos. 2-290812 and 6-62795). Patent applications for use of EPA or the like as an external preparation also have been filed (Japanese Unexamined Patent Application Publication Nos. 3-90046, 8-109128, 6-298642, and 7-112913), but they have not been put in practical use. Japanese Unexamined Patent Application Publication 5-58902 has disclosed fat and oil compositions for preventing allergies containing EPA or the like and sesame oil. These compositions are also capsulated for internal applications; hence they do not have such antioxidant properties as to be used in external application.

In order to enhance the oxidative stability of oils and fats, various types of antioxidant have been used. For example, plural types of antioxidant are used in combination, or a synergist, such as phosphoric acid, citric acid, or ascorbic acid, is added to an antioxidant to enhance the antioxidant properties. These approaches are effective for vegetable oils or the like (whose fatty acids have 3 or less double bonds). However, the oxidation stabilities of fish oils and other oils and fats having extremely low oxidative stability (whose fatty acids have at least four double bonds) cannot be sufficiently enhanced by only such combinations of antioxidants and synergists.

Sesame oil is relatively stable to oxidation, and it has been known since a long time ago that sesame contains antioxidative components, such as sesamol and other lignans (Japanese Unexamined Patent Application Publication No. 58-132076; Shoku no Kagaku, 225 (11) pp. 40-48 (1996); Shoku no Kagaku, 225 (11) pp. 32-36 (1996)).

Sesamol is an antioxidant for oils and fats and food containing oil or fat, and is approved as a food additive. It is however reported that sesamol is not effective for oils and fats exhibiting extremely low oxidative stability, such as fish oil (NOF Corporation, from fiscal Heisei 4 (1994) to Heisei 8 (1996), DHA Koudo Seisei Chushutsu Gijutsu Kaihatsu Jigyo, Kekka Gaiyou (DHA Koudo Seisei Chushutsu Gijutsu Kenkyu Kumiai) pp. 74-79 (2002)). Sesamol is not used for enhancing the oxidative stability of fish oil.

Ascorbic acid and ascorbic acid derivatives are also approved as food additives and used as antioxidants for oils and fats and food containing oil or fat. However, they are not effective for oils and fats exhibiting extremely low oxidative stability, such as fish oil, if they are used alone, and even their combined use with tocopherol does not produce satisfactory effects.

In order to prevent the oxidation of oils and fats, combined use of various types of antioxidant has been attempted. For example, Japanese Unexamined Patent Application Publication No. 2002-142673 has disclosed a lipophilic antioxidant prepared by emulsifying gallic acid, a water-soluble antioxidant, and an oil-soluble antioxidant into a water-in-oil form with a lipophilic emulsifier. In this application, examples of the water-soluble antioxidant include vitamin C, citric acid, chlorogenic acid, their derivatives, sugar-amino reaction products, proanthocyanidin, flavone derivatives, tea extracts, grape seed extracts, and rutin, and examples of the oil-soluble antioxidant include tocopherol, ascorbyl palmitate, sesamol, and γ-oryzanol.

Effects of antioxidants have been compared for pyrolysis of tocopherol in vegetable oils in Nippon Eiyo Shokuryo Gakkaishi (Journal of Japanese Society of Nutrition and Food Science), 44 (6) pp. 493-498 (1991), 45 (3) pp. 291-295 (1992), and 45 (3) pp. 285-290 (1992). Although some of the antioxidants use sesamol and an ascorbic acid ester in combination, they do not produce effects particularly superior to other antioxidants. These literatures discuss effects in oxidation of vegetable oils (having 3 or less unsaturated bonds) at high temperatures, but not in oxidation of polyunsaturated fatty acids (having at least three unsaturated bonds) during storage at room temperature; hence different objects are used under different conditions. This is probably because the combination of sesamol and an ascorbic acid ester does not produce superior effects. In addition, the thermal instability of the antioxidants may affect antioxidant properties.

As described above, although sesamol and an ascorbic acid or its ester are well known as antioxidants, it has not been known that their combination produces antioxidative effects particularly superior to other antioxidants.

Since external preparations are spread on the surface of skin to come into contact with oxygen at a large area, they are liable to stand in a state to be oxidized and are, in addition, allowed to stand in such a state for a long time. Therefore, external preparations require higher oxidative stability than internal medicines, for practical use. Also, stains of EPA or the like on clothing are difficult to remove by normal washing, and their odor remains for a long time. EPA and the like have such a disadvantage in using as drugs in practice, in spite of their efficacy. Furthermore, it has generally known that peroxides produced by oxidation of EPA or the like harmfully affect living bodies. To suppress the production of peroxides is one of the important objects.

Conventional external preparations contain synthetic antioxidants, such as t-butylhydroxytoluene (BHT), butylhydroxyanisole (BHA), t-butylhydroquinone (TBHQ), and ethoxyquin.

### DISCLOSURE OF INVENTION

The object of the present invention is to provide an external composition containing a polyunsaturated fatty acid or its salt or ester, exhibiting extremely enhanced oxidative stability. Specifically, the present invention provides an external composition containing a polyunsaturated fatty acid, such as EPA, and which does not generate odors or peroxides resulting from oxidation when it is applied to skin.

The inventors of the present invention have conducted intensive research in order to enhance the oxidative stability of oils and fats (oils and fats containing a high proportion of polyunsaturated fatty acid, such as oils of fish and aquatic animals). As a result, the inventors found that the oxidative stability of oils and fats can be extremely enhanced by adding an antioxidative component of sesame, such as sesamol, an ascorbic acid or ascorbyl fatty acid ester, and tocopherol in combination, and thus accomplished the present invention.

The main points of the present invention are the external composition and its production process described in the following (1) to (15).
(1) An external composition having oxidative stability comprising: a polyunsaturated fatty acid or its salt or ester which contains an antioxidant comprising an antioxidative sesame component and ascorbic acid or an ascorbyl fatty acid ester; and an external base material.
(2) An external composition having oxidative stability comprising: a polyunsaturated fatty acid or its salt or ester which contains an antioxidant comprising an antioxidative sesame component and ascorbic acid or an ascorbyl fatty acid ester; and an external base material, wherein the antioxidative sesame component is at least one of the substances represented by peaks detected by high-performance liquid chromatography using an electrochemical detector at elution times of about 2.66, 3.40, 3.84, 4.57, 4.98, 5.82, 7.00, 8.67, 9.84, 11.24, 12.29, 12.49, 13.36, 14.04, 14.32, 14.74, 15.22, 15.60, 15.82, 16.34, 16.98, 18.10, 18.43, and 34.91 minutes.
(3) An external composition having oxidative stability comprising: a polyunsaturated fatty acid or its salt or ester which contains an antioxidant comprising an antioxidative sesame component and ascorbic acid or an ascorbyl fatty acid ester; and an external base material, wherein the antioxidative sesame component is extracted from sesame, sesame oil, or sesame residue, using a solvent, a lipid, or an emulsifier singly or in combination.
(4) An external composition having oxidative stability comprising: a polyunsaturated fatty acid or its salt or ester which contains an antioxidant comprising an antioxidative sesame component and ascorbic acid or an ascorbyl fatty acid ester; and an external base material, wherein the antioxidative sesame component is at least one selected from the group consisting of sesamol, sesaminol, episesaminol, pilsinol, epihinoresinol, syringaresinol, samine, sesamolinol, and 2,3-di(4'-hydroxy-3'-methoxybenzyl)-2-buten-4-olide.
(5) The external composition according to any one of (1) to (4), wherein the ascorbyl fatty acid ester contains ascorbyl palmitate or ascorbyl stearate.
(6) The external composition according to any one of (1) to (4), wherein the ascorbic acid or the ascorbyl fatty acid ester is contained in an excessive amount more than the amount soluble in the polyunsaturated fatty acid or its salt or ester.
(7) The external composition according to (1) to (4), wherein the excessive amount of the ascorbic acid or the ascorbyl fatty acid ester is in a powder or solid form.
(8) The external composition according to any on of (1) to (7), wherein the polyunsaturated fatty acid contains at least one of eicosapentaenoic acid and docosahexaenoic acid.
(9) The external composition according to any one of (1) to (8), wherein the ester of the polyunsaturated fatty acid is a triglyceride containing the polyunsaturated fatty acid as a constituent, or a lower alcohol ester of the polyunsaturated fatty acid.
(10) The external composition according to any one of (1) to (8), wherein the ester of the polyunsaturated fatty acid is added in a form of refined fish oil.
(11) The external composition according to any one of (1) to (10), further comprising tocopherol.
(12) The external composition according to any one of (1) to (11), wherein the external composition is used for a skin inflammatory disease.
(13) The external composition according to any one of (1) to (12), wherein the external base material is an oleaginous base material such as white ointment, white petrolatum, or zinc oxide ointment; or an emulsion base material (cream), such as hydrophilic ointment, purified lanolin, water-absorbable ointment, or urea cream.
(14) The external composition according to (13), wherein the external base material is a white ointment, a white petrolatum, a urea cream, or a zinc oxide ointment.
(15) The method for producing an external composition having oxidative stability, containing a polyunsaturated fatty acid or its salt or ester, wherein an antioxidative sesame component, ascorbic acid or an ascorbyl fatty acid ester, and an external base material are mixed with the polyunsaturated fatty acid or its salt or ester, or an oil or fat containing the polyunsaturated fatty acid or its salt or ester, under conditions preventing oxygen from mixing, and the mixture is quickly placed in a hermetic container, and wherein the polyunsaturated fatty acid or its salt or ester has an peroxide value (PV) of 3.0 meq/kg or less and an acid value (AV) of 1.0 or less, and no odor in terms of sensory testing.
(16) The method according to (15), wherein the conditions preventing oxygen from mixing is an atmosphere purged with an inert gas.

The present invention provides an external composition exhibiting oxidative stability extremely superior to the known external compositions containing a polyunsaturated fatty acid or the like. The present invention can also provide external medical drugs, external quasi drugs, and cosmetic preparations containing a polyunsaturated fatty acid or the like, such as EPA, which has not been put into practical use as external preparations due to an odor resulting from oxidation, in spite of its efficacy. Since the composition of the present invention exhibits superior oxidative stability, it can be easily produced and preserved even though such a polyunsaturated fatty acid is used. Also, the polyunsaturated fatty acid content can be increased.

### BRIEF DESCRIPTION OF THE DRAWINGS

Fig. 1 shows amounts of oxygen absorbed by oil or fat in Experimental Example 1.
Fig. 2 shows amounts of oxygen absorbed by oil or fat in Experimental Example 2.
Fig. 3 shows amounts of oxygen absorbed by oil or fat in Experimental Example 3.
Fig. 4 shows amounts of oxygen absorbed by oil or fat in Experimental Example 4.
Fig. 5 shows amounts of oxygen absorbed by oil or fat in Experimental Example 5.
Fig. 6 is a high-performance liquid chromatographic chart of sesame residue extract 1, obtained with an electrochemical detector in Experimental Example 6.
Fig. 7 shows changes with time in the amount of oxygen absorbed by samples of Experimental Example 6.
Fig. 8 shows changes with time in the malondialdehyde content in samples of Experimental Example 7.
Fig. 9 shows changes with time in the alkenal content in samples of Experimental Example 7.
Fig. 10 shows changes with time in the amounts of oxygen absorbed by a sample (sesamol, 0.5%) and the remaining amounts of antioxidants in Experimental Example 8.
Fig. 11 shows changes with time in the amounts of oxygen absorbed by a sample (sesamol, 1.0%) and the remaining amounts of antioxidants in Experimental Example 8.
Fig. 12 shows changes with time in the amounts of oxygen absorbed by samples and the remaining amounts of antioxidants in the case where ascorbyl palmitate was added after 4 days in Experimental Example 8.
Fig. 13 shows changes with time in the PV of samples of Experimental Example 9.
Fig. 14 shows changes with time in the amount of oxygen absorbed by samples of Fig. 13.

### BEST MODE FOR CARRYING OUT THE INVENTION

The polyunsaturated fatty acid or its salt or ester used herein refers to a polyunsaturated fatty acid, a lower alcohol ester of the polyunsaturated fatty acid, and a triglyceride containing the polyunsaturated fatty acid as a constituent. Its examples include oils of fish and aquatic animals containing a high proportion of eicosapentaenoic acid, docosahexaenoic acid, or the like, and their esters, such as eicosapentaenoic acid ethyl ester and docosahexaenoic acid ethyl ester. The polyunsaturated fatty acid refers to a fatty acid having 3 or more double bonds. Exemplary polyunsaturated fatty acids having 3 or more double bonds include α-linolenic acid, arachidonic acid, eicosapentaenoic acid, and docosahexaenoic acid. Also, polyunsaturated fatty acid-based compounds used in the present invention include esters of such fatty acids, such as methyl esters, ethyl esters, triglycerides, ditriglycerides, and monoglycerides.

Eicosapentaenoic acid is a generic name of fatty acids having a carbon number of 20 and five double bonds, and natural eicosapentaenoic acids are cis-type pentavalent straight-chain unsaturated n-3 fatty acids having double bonds at the 5, 8, 11, 14, and 17 positions. Docosahexaenoic acid is a straight-chain hexenoic acid having a carbon number of 22 and cis-double bonds at the 4, 7, 10, 13, 16, and 19 positions. These EPAs and DHAs derived from nature are contained in natural oils and fats, particularly in oils and fats of marine products, such as tuna, bonito, chub mackerel, sardine, and pacific cod. They may be present in a form of glyceride or other derivatives.

In the present invention, any material can be used as long as it results in an oil or fat containing a polyunsaturated fatty acid having 3 or more double bonds. Examples of the material for oil or fat containing the polyunsaturated fatty acid include marine fish, such as sardine, chub mackerel, saury, tuna, and bonito; and fats derived from microorganisms; crustaceans, such as euphausiid and shrimp and lobster; fish oil; animal and vegetable oils; and genetically modified vegetable oils.

The polyunsaturated fatty acid having 3 or more double bonds can be concentrated by wintering or enzymatically treating oil or fat containing the polyunsaturated fatty acid. Alternatively, the oil or fat containing a polyunsaturated fatty acid having 3 or more double bonds may be esterified with alcohol or hydrorified to fatty acid, and then subjected to distillation, urea addition, column treatment, enzymatic treatment, or supercritical carbon dioxide treatment. Thus, the polyunsaturated fatty acid can be concentrated.

The antioxidant used in the present invention does not reduce substances that have been already oxidized. It is therefore necessary that oxides be removed from the polyunsaturated fatty acid or its salt or ester to be used, by degumming, deacidification, decolorization, deodorization, or the like before use of the antioxidant. Preferably, the polyunsaturated fatty acid or its salt or ester is purified to a PV of 3.0 meq/kg or less and an AV of 1.0 or less, and has no odor in terms of sensory testing.

The oil or fat constituted of the polyunsaturated fatty acid, which is contained in the external preparation of the present invention, may be acidified by hydrolysis or oxidation. The oxidation produces decomposition products of hydroperoxides. For example, soybean oil is oxidized to produce propionaldehyde, 2-pentenal, caproic aldehyde, acetaldehyde, and crotonaldehyde. These oxidation products from the polyunsaturated fatty acid are causes of disagreeable odors of fish oil, and thus a fishy odor peculiar to fish oil is generated. Refined oils containing the unsaturated fatty acid, such as fish oil, soybean oil, linseed oil, and rape-seed oil, may generate disagreeable odor or change in color in the very early stages of their oxidation. This phenomenon is called "Modori" (deterioration). The "Modori" phenomenon in color of decolorized refined vegetable oil is caused by an oxidation product from vitamin E, chromane-5,6-quinone. The present invention is intended to prevent the polyunsaturated fatty acid from oxidizing, and produces remarkable effects particularly in animal oil having a low oxidative stability.

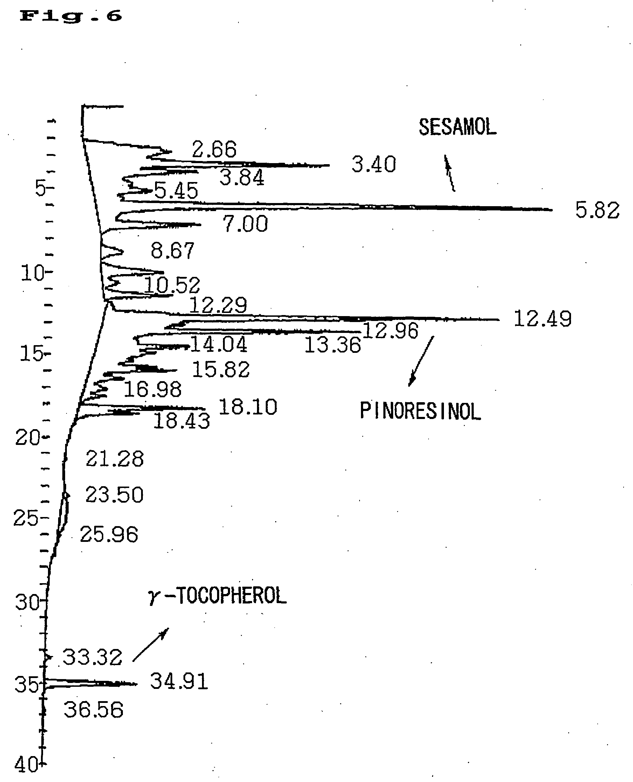

The antioxidative sesame component used in the present invention may be of phenol form. Examples such antioxidative components include sesamol, sesaminol, episesaminol, pilsinol, epihinoresinol, syringaresinol, samine, sesamolinol, and 2,3-di(4'-hydroxy-3'-methoxybenzyl)-2-buten-4-olide. In Fig. 6, the peaks of the HPLC chart, which were detected by an electrochemical detector, represent their respective antioxidative components. This chart shows that sesame contains many antioxidative components. While these components, including sesamol, can produce a satisfactory effect independently, mixtures of these components exhibit stronger antioxidant properties. The antioxidative sesame components may be used singly or in combination.

Hence, the antioxidative sesame component used in the present invention may be highly purified antioxidative component from sesame, or a lightly purified antioxidative component including sesamol, which is also containing the other substance derived from sesame, such as sesame lignan, or tocopherol. The antioxidative sesame component may be synthesized. Furthermore, sesame oil may be used as it is, as long as there is no problem with the odor peculiar to sesame oil.

Specifically, the antioxidative component can be extracted from sesame seeds, sesame oil, or degreased sesame residue after expressing sesame oil. Alternatively, it can be obtained from scum, which is a component prepared by distillation during deodorization of sesame oil. Preferably, roasted sesame is used because the antioxidative component is increased by roasting. Since non-roasted sesame contains a certain proportion of antioxidative component, it can also be used. For extraction from sesame seeds, it is preferable that the principal constituent or neutral lipid be expressed, or removed with a nonpolar solvent, such as hexane. Although sesame oil can be used as it is, its application is limited because the absolute quantity of the neutral lipid is high and, accordingly, the antioxidative component content is low.

The sesame extract used in the present invention may be prepared by any method, as long as antioxidative components such as represented by HPLS peaks shown in Fig. 6 can be extracted. For example, the antioxidative components can be extracted by use of a solvent, a lipid, or an emulsifier. More specifically, the antioxidative components can be extracted from sesame, sesame oil, or sesame residue by use of organic solvents, such as nitrous oxide, acetone, ethanol, ethyl methyl ketone, glycerol, ethyl acetate, methyl acetate, diethyl ether, cyclohexane, dichloromethane, 1,1,1,2-tetrafluoroethane, 1,1,2-trichloroethane, carbon dioxide, 1-butanol, 2-butanol, butane, 1-propanol, 2-propanol, propane, propylene glycol, hexane, and methanol; lipids, such as triglyceride, diglyceride, and monoglyceride; and emulsifiers, such as propylene glycol fatty acid esters, polyglycerol fatty acid esters, and sorbitan fatty acid esters. In addition, after removal of the solvent by evaporation, the extract is redissolved in an organic solvent. Then, water-soluble constituents are removed by partition with water, or insoluble constituents are removed by filtration. Thus, the antioxidative component can be concentrated.

In the present invention, the amount of antioxidant used in the external composition can be varied depending on the storage conditions and period or the base material used. If sesamol of sesame is used as the antioxidant, a content of 0.5% or more to the polyunsaturated fatty acid is effective. Although at most about 0.1% of ascorbyl fatty acid ester can be dissolved in the polyunsaturated fatty acid, it can be appropriately increased according to the application because the duration of antioxidant properties can be,enhanced by adding an excessive amount of the antioxidant. For example, an external composition containing 5% of polyunsaturated fatty acid added 1.0% of sesamol and 0.5% of ascorbyl palmitate can provide a product capable of being preserved for one year at room temperature.

Examples of the ascorbic acid or ascorbyl fatty acid ester used in the present invention include ascorbic acid and ascorbyl fatty acid esters such as ascorbyl palmitate and ascorbyl stearate. As an alternative to these materials, salts of ascorbic acid can also be used. Preferred are materials having a high solubility in lipids.

In general, when an antioxidant is added to oil or fat, a soluble amount is added. This is because a product containing insoluble matter resulting from excessive addition is considered to be a defective contaminated with foreign matter, and because it has not been considered that excessive addition of the antioxidant produces effects, unless it is dissolved.

On the other hand, the present inventors found a relationship in content between an antioxidative sesame component and ascorbic acid or an ascorbyl fatty acid ester. Specifically, the presence of an excessive amount of ascorbyl fatty acid ester maintains the antioxidant properties longer even if the content of sesamol, an antioxidative sesame component, is constant, as shown in Experimental Examples 11 and 12. The excessive ascorbic acid or ascorbyl fatty acid ester in the mixture may be in powder form or solid. However, if a viscous base material is used, the ascorbic acid or ascorbyl fatty acid ester needs to be fine powder in order to be uniformly mixed because natural diffusion in such a base material becomes slow. It goes without saying that use of coarse powder is not favorable because external preparations using such powder have a bad feel when it is used.

The oil or fat whose oxidative stability has been enhanced by adding an antioxidative sesame component and ascorbic acid or an ascorbyl fatty acid ester can exhibit satisfactory oxidative stability alone, but it may be used in combination with another antioxidant. Also, it may be mixed with another oil or fat having superior oxidative stability (for example, vegetable oil).

In addition to the antioxidative sesame component and the ascorbic acid or ascorbyl fatty acid ester, a tocopherol may be added. The tocopherol may be selected from among α-, β-, γ-, and δ-tocopherols and mixed tocopherol, but preferably δ-tocopherol is used. Tocopherol is often added to commercially available polyunsaturated fatty acids and their salts and esters, such as refined fish oil, in the stage of production. Hence, use of these raw materials naturally results in a product containing tocopherol. Whether tocopherol is present or absent does not affect the synergistic effect of the antioxidative sesame component and the ascorbic acid or ascorbyl fatty acid ester.

The content of docosahexaenoic acid, eicosapentaenoic acid, or their ester in the external composition of the present invention is not particularly limited. The content can be set according to the application. The lower limit refers to a minimum amount capable of producing the effect, and the upper limit can be set at an amount capable of being dissolved in a selected base material. Specifically, the content in the external preparation is normally in the range of 0.01% to 20%.

The external medical drug, external quasi drug, and cosmetic preparation provided by the present invention are not particularly limited in form, as long as their active constituents can be directly applied onto a local surface of skin. Examples of the external preparation include ointments, such as oleaginous ointment, emulsion ointment (cream), and water-soluble ointment, gel, lotion, tapes, adhesive preparations, spray, gel, and reservoir-type patches.

The external composition of the present invention may contain additives generally used in external compositions, such as a base material, an absorption promoter, a moisturizing agent, a thickener, an emulsifier, a colorant, an aromatic substance, an antioxidant, a stabilizing agent, a fungicide, and an antiseptic, if necessary.

Exemplary base materials include white petrolatum, cetanol, stearyl alcohol, stearic acid, bleached beeswax, liquid paraffin, lauromacrogol, squalane, squalene, lanoline, isobutyl myristate, and medium-chain triglycerides. Since the polyunsaturated fatty acid or its salt or ester is oleaginous, it can be uniformly mixed with the oleaginous base material. If the base material contains water, it can be easily mixed with oil by being emulsified into a W/O type or an O/W type and adding the emulsified base material to the oil.

Exemplary absorption promoters include urea, crotamiton, diethyl sebacate, and diisopropyl adipate.

Exemplary moisturizing agents include polyhydric alcohols such as glycerol, sorbitol, propylene glycol, 1,3-butanediol, sodium pyrrolidone carboxylate, and sodium hyaluronate.

Exemplary thickeners include gum alabic, guar gum, locust bean gum, carrageenan, carboxymethyl cellulose, sodium carboxymethyl cellulose, salts of polyacrylic acids, polyacrylic acid esters, natural latex, vinyl acetate resin emulsion, polyvinyl alcohol, hydroxypropyl cellulose, and hydroxypropyl methylcellulose.

Exemplary emulsifiers include glycerol fatty acid esters, sucrose fatty acid esters (sugar esters), sorbitan fatty acid esters, propylene glycol fatty acid esters, lecithin, polyoxyethylene fatty acid esters, polyoxyethylene castor oil, polyoxyethylene hydrogenated castor oil, polyoxyethylene alkyl ethers, polyoxyethylene sorbitan fatty acid esters, and sodium lauryl sulfate.

Exemplary aromatic substances include eucalyptus oil, lavender oil, menthol, peppermint oil, rose oil, orange oil, cherry flavor, fruit flavor, vanillin, and vanilla flavor.

Exemplary antioxidants include L-ascorbic acid, ascorbyl stearate, ascorbyl palmitate, sodium ascorbate, erythorbic acid, sodium erythorbate, isopropyl citrate, dl-α-tocopherol, dl-δ-tocopherol, dibutylhydroxytoluene (BHT), and butylhydroxyanisole (BHA). Among these preferred are ascorbyl palmitate, ascorbyl stearate, L-ascorbic acid, dl-α-tocopherol, and dl-δ-tocopherol. Combinations of these antioxidants are also preferable.

Exemplary stabilizing agents include polysorbate, polyethylene glycol, ethanol, acetone, light anhydrous silicic acid, and EDTA.

Exemplary antiseptics include p-hydroxybenzoate esters, benzalkonium chloride, sorbic acid, phenol, chlorobutanol, chlorocresol, and benzyl alcohol.

The external composition of the present invention may further contain medicinal properties of conventionally used drugs for curing dermatitis or allergodermia, such as steroid, antiallergic drugs, immunosuppressive drugs, and antiinflammatory drugs.

The external composition of the present invention is effective for dermatitis and allergodermia for which polyunsaturated fatty acids are effective. Specifically, the external composition can be directly applied to areas affected by, for example, atopic dermatitis or psoriasis.

The amount of the external composition to be administered can be widely varied depending on the symptom, affected area, age, weight, how the drug is administered, and other factors. In general, the normal amount is in the range of 0.1 to 5 g/day. Such an amount is generally applied directly to the affected area at one time or several times.

Although the external composition of the present invention has superior oxidative stability, it is necessary to prevent the polyunsaturated fatty acid from oxidizing, and to avoid placing the polyunsaturated fatty acid in an excessively oxidizing environment, in the production process. Otherwise, the effect of the external composition cannot be taken advantage of.

The antioxidant may be added to the polyunsaturated fatty acid or its salt or ester in advance, and then mixed with the base material, or the three constituents may be mixed at one time. In either case, it is necessary to prevent the polyunsaturated fatty acid from oxidizing as much as possible. Preferably, all the steps in the production are performed in an atmosphere purged with an inert gas, such as nitrogen, helium, or argon.
Alternatively, the steps may be performed in an apparatus preventing air from mixing.

Exemplary formulas of the external composition of the present invention are as follows:
(1) Oleaginous Base Material
[White Ointment]

**Table 1**

| Constituent | Content in 100 g |
|---|---|
| Refined fish oil | 4.9 g |
| δ-Tocopherol | 0.025 g |
| Sesamol | 0.05 g |
| Ascorbyl palmitate | 0.025 g |
| Bleached beeswax | 4.75 g |
| Sorbitan sesquioleate | 1.9 g |
| White petrolatum | balance |

[White Petrolatum]

**Table 2**

| Constituent | Content in 100 g |
|---|---|
| Refined fish oil | 4.9 g |
| δ-Tocopherol | 0.025 g |
| Sesamol | 0.05 g |
| Ascorbyl palmitate | 0.025 g |
| White petrolatum | 95.0 g |

(2) Emulsion Base Material
[Hydrophilic ointment ]

**Table 3**

| Constituent | Content in 100 g |
|---|---|
| Refined fish oil | 4.9 g |
| δ-Tocopherol | 0.025 g |
| Sesamol | 0.05 g |
| Ascorbyl palmitate | 0.025 g |
| White petrolatum | 23.75 g |
| Stearyl alcohol | 19.0 g |
| Propylene glycol | 11.4 g |
| Polyoxyethylene hydrogenated castor oil 60 | 3.8 g |
| Glyceryl monostearate | 0.95 g |
| Methyl p-hydroxybenzoate | 0.095 g |
| Propyl p-hydroxybenzoate | 0.095 g |
| Purified water | balance |

[Purified Lanolin]

**Table 4**

| Constituent | Content in 100 g |
|---|---|
| Refined fish oil | 4.9 g |
| δ-Tocopherol | 0.025 g |
| Sesamol | 0.05 g |
| Ascorbyl palmitate | 0.025 g |
| Purified lanolin | 95.0 g |

[Water-Adsorbable Ointment]

**Table 5**

| Constituent | Content in 100 g |
|---|---|
| Refined fish oil | 4.9 g |
| δ-Tocopherol | 0.025 g |
| Sesamol | 0.05 g |
| Ascorbyl palmitate | 0.025 g |
| White petrolatum | 38.0 g |
| Cetyl alcohol | 9.5 g |
| Bleached beeswax | 4.75 g |
| Sorbitan sesquioleate | 4.75 g |
| Lauromacrogol | 0.475 g |
| Methyl p-hydroxybenzoate | 0.095 g |
| Propyl p-hydroxybenzoate | 0.095 g |
| Purified water | balance |

(3) Zinc Oxide Ointment

**Table 6**

| Constituent | Content in 100 g |
|---|---|
| Refined fish oil | 4.9 g |
| δ-Tocopherol | 0.025 g |
| Sesamol | 0.05 g |
| Ascorbyl palmitate | 0.025 g |
| Zinc oxide | 19.0 g |
| Liquid paraffin | 2.85 g |
| White ointment | balance |

(4) Urea Cream

**Table 7**

| Constituent | Content in 100 g |
|---|---|
| Refined fish oil | 4.9 g |
| δ-Tocopherol | 0.025 g |
| Sesamol | 0.05 g |
| Ascorbyl palmitate | 0.025 g |
| Urea | 4.75 g |
| Cetyl alcohol | 4.75 g |
| Glyceryl monostearate | 4.75 g |
| Liquid paraffin | 4.75 g |
| Glycerol | 9.5 g |
| Polyoxyethylene 20 cetyl ether | 2.375 g |
| Methyl polysiloxane | 0.95 g |
| Methyl p-hydroxybenzoate | 0.19 g |
| Tetrasodium edetate | 0.19 g |
| Sodium citrate | 0.19 g |
| Purified water | balance |

### EXAMPLES

The present invention will be further described with reference to the following examples, but the invention is not limited to the examples.

In the examples, the following materials were used as the refined fish oil, sesamol, ascorbyl palmitate, ascorbic acid, δ-tocopherol, eicosapentaenoic acid ethyl ester, and external base material.

Refined fish oil (containing 0.5% by weight of δ-tocopherol): DD Oil Type 3 (refined fish oil produced by refining tuna oil to a peroxide value of 5 meq/kg or less, an acid value of 1 or less, and a color Gardner of 3 or less by degumming, deacidification, deodorization, or other process; containing 8% by weight of EPA and 22% by weight of DHA), produced by Nippon Suisan Kaisha, Ltd.

Refined fish oil (not containing δ-tocopherol): taken as a sample before adding δ-tocopherol in the process of DD Oil Type 3 preparation, produced by Nippon Suisan Kaisha, Ltd.

Refined fish oil (sardine oil): DD Oil Type 2 (refined fish oil produced by refining sardine oil to a peroxide value of 5 meq/kg or less, an acid value of 1 or less, and a color Gardner of 3 or less by degumming, deacidification, deodorization, or other process; containing 28% by weight of EPA, 12% by weight of DHA, and 0.5% by weight of δ-tocopherol), produced by Nippon Suisan Kaisha, Ltd.

Sesamol: sesamol (purity: 98%) produced by Nacalai Tesque, Inc.

Ascorbyl palmitate: ascorbyl palmitate (purity: 95% or more) produced by Sankyo Foods Co. Ltd.

Ascorbic acid: L(+)-ascorbic acid (purity: 99.5%) produced by Nacalai Tesque, Inc.

δ-Tocopherol: D-δ-tocopherol (purity: 90%) produced by Wako Pure Chemical Industries

Eicosapentaenoic acid ethyl ester: prepared by ethanolysis of sardine oil in the presence of metallic sodium to prepare sardine oil ethyl ester and purifying the ester by distillation and HPLC to a purity of 99%.

α-Tocopherol: (±)-α-tocopherol (purity: 98%) produced by Wako Pure Chemical Industries

External base material or its constituents: bleached beeswax (Kobayashi Kako Co., Ltd.), sorbitan sesquioleate (Wako Pure Chemical Industries), white petrolatum (Wako Pure Chemical Industries), white petrolatum (Wako Pure Chemical Industries), hydrophilic ointment (Nikko Seiyaku), purified lanolin (Nikko Seiyaku), water-adsorbable ointment (Nikko Seiyaku), zinc oxide (Wako Pure Chemical Industries), liquid paraffin (Junsei Chemical Co., Ltd.), urea (Junsei Chemical Co., Ltd.), cetyl alcohol (Kyowa Tecnos Co., Ltd.), glyceryl monostearate (Tokyo Kasei Kogyo Co., Ltd.), liquid paraffin, (Junsei Chemical Co., Ltd), glycerol (Wako Pure Chemical Industries), polyoxyethylene (20) cetyl ether (Wako Pure Chemical Industries), methyl polysiloxane (Tokyo Kasei Kogyo Co., Ltd.), methyl p-hydroxybenzoate (Tokyo Kasei Kogyo Co., Ltd.), tetrasodium edetate (Dojindo Laboratories), sodium citrate (Wako Pure Chemical Industries)

### EXAMPLES 1 to 7

### <Preparation of External Compositions>

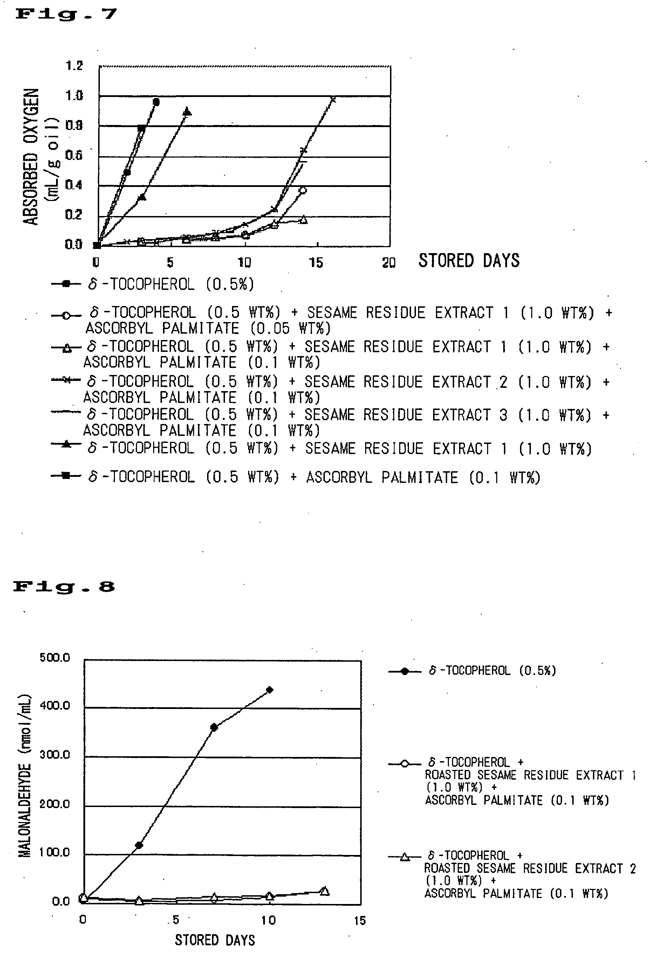

Refined fish oil and an antioxidant were added to each external base material shown in Fig. 8 to prepare external compositions. The compositions of Examples 1 to 7 were shown in Tables 1 to 7, respectively. Specifically, 5 g of refined fish oil (sardine oil) containing 0.5% by weight of δ-tocopherol, mixed with the antioxidant preparation of the present invention (1.0% of sesamol + 0.5% of ascorbyl palmitate) was slowly added to 95 g of each of the prepared base materials in a nitrogen atmosphere while being mixed in a glass mortar. The resulting mixture was quickly preserved in an aluminium tube in a nitrogen atmosphere. For comparative examples, only the refined fish oil (sardine oil) containing 0.5% by weight of δ-tocopherol was added to each base material.

**Table 8**

| | Base material | Classification 1 | Classification 2 | Classification 3 |
|---|---|---|---|---|
| Example 1 | White petrolatum | Hydrophobic | Oleaginous | Mineral |
| Example 2 | White ointment | | | |
| Example 3 | Zinc oxide ointment | | | |
| Example 4 | Purified lanolin | Hydrophilic | Emulsion | W/O (no water phase) |
| Example 5 | Hydrophilic ointment | | | O/W |
| Example 6 | Urea cream | | | |
| Example 7 | Water-adsorbable ointment | | | W/O (having water phase) |

### <Antioxidant Property Test of External Compositions>

The storage stability of each external composition prepared in the above manner was examined.

Each external composition preserved in the aluminum tube was stored at 40°C. The stored external composition was evaluated by sensory test of the degree of fishy odor and by chemical analysis of oxidation, after storage for 2 weeks, 1 month, 2 months, and 3 months.

### [Sensory Test Method]

A certain amount of external composition was applied onto the skin of the hands of three panelists specializing in fishy odor sensory test, and the degree of the fishy odor was evaluated immediately after the application and after 0 to 3 hours according to the evaluation criteria shown in Table 9.

**Table 9**

| Point | Fishy odor level |
|---|---|
| 0 | No fishy odor |
| 1 | Very little fishy odor |
| 2 | Slight fishy odor |
| 3 | Distinct fishy odor |
| 4 | Disagreeable fishy odor |
| 5 | Unbearable fishy odor |

### Results

The results of the sensory test are shown in Table 10 (immediately after the application) and Table 11 (samples stored at 40°C for 2 months, 0 to 3 hours after the application).

**Table 10**

| | | After 2 weeks | After 1 month | After 2 months | After 3 months |
|---|---|---|---|---|---|
| White petrolatum | Example 1 | 0.0±0.0 | 0.0±0.0 | 0.3±0.5 | 0.0±0.0 |
| | Comparative Example 1 | 0.0±0.0 | 0.0±0.0 | 2.7±0.9 | 4.0±0.0 |
| White ointment | Example 2 | 0.0±0.0 | 0.0±0.0 | 0.0±0.0 | 0.0±0.0 |
| | Comparative Example 2 | 0.7±0.5 | 0.7±0.5 | 0.3±0.5 | 2.7±0.5 |
| Zinc oxide ointment | Example 3 | 0.0±0.0 | 0.0±0.0 | 0.0±0.0 | 0.0±0.0 |
| | Comparative Example 3 | 1.0±0.8 | 1.0±0.8 | 0.0±0.0 | 0.3±0.5 |
| Purified lanolin | Example 4 | 0.0±0.0 | 0.0±0.0 | 0.0±0.0 | 0.0±0.0 |
| | Comparative Example 4 | 0.0±0.0 | 0.0±0.0 | 0.3±0.5 | 0.0±0.0 |
| Hydrophilic ointment | Example 5 | 0.3±0.5 | 0.3±0.5 | 2.7±0.5 | 2.3±0.5 |
| | Comparative Example 5 | 3.7±0.0 | 3.7±0.5 | 4.3±0.5 | 3.7±0.5 |
| Urea cream | Example 6 | 0.0±0.0 | 0.0±0.0 | 0.0±0.0 | 0.0±0.0 |
| | Comparative Example 6 | 3.0±0.8 | 3.3±0.5 | 3.7±0.5 | 5.0±0.0 |
| Water-adsorbable ointment | Example 7 | 0.0±0.0 | 0.0±0.0 | 0.0±0.0 | 0.0±0.0 |
| | Comparative Example 7 | 0.3±0.5 | 0.0±0.0 | 0.0±0.0 | 0.0±0.0 |

**Table 11**

| | | Immediately after | After 0.5 hour | After 1 hour | After 2 hours | After 3 hours |
|---|---|---|---|---|---|---|
| White ointment | Example 2 | 0.0±0.0 | 0.0±0.0 | 0.0±0.0 | 0.0±0.0 | 0.0±0.0 |
| | Comparative Example 2 | 0.3±0.5 | 0.7±0.5 | 1.3±0.5 | 1.7±0.5 | 1.0±0.8 |
| Zinc oxide ointment | Example 3 | 0.0±0.0 | 0.0±0.0 | 0.3±0.5 | 0.3±0.5 | 0.7±0.5 |
| | Comparative Example 3 | 0.3±0.5 | 2.0±1.4 | 2.3±1.3 | 3.0±0.8 | 3.0±0.8 |

For the samples using the white petrolatum as the base material, Comparative Example 1 generated a fishy odor after storage at 40°C for 2 months, and the fishy odor was stronger after storage at 40°C for 3 months. In contrast, Example 1 was able to substantially completely prevent the generation of fishy odor even when it had been stored at 40°C for 3 months.

For the samples using the white ointment as the base material, Comparative Example 2 generated a fishy odor after storage at 40°C for 3 months, but not after storage at 40°C for 2 months. In contrast, Example 2 was able to substantially completely prevent the generation of fishy odor even when it had been stored at 40°C for 3 months. In Table 11, while Comparative Example 2 generated a fishy odor in 2 hours after the application, Example 2 did not even in 3 hours after the application.

For the samples using the zinc oxide ointment as the base material, Comparative Example 3 stored at 40°C slightly generated a fishy odor until one month had elapsed, but there was no odor after that. The sample of Example 3 was able to substantially completely prevent the generation of fishy odor even when it had been stored at 40°C for 3 months. In the comparisons with time after the application, shown in Table 11, Comparative Example 3 generated a fishy odor in 0.5 hour after the application, and the odor became stronger with time. In contrast, Example 3 hardly generated fishy odor over 3 hours after the application.

In Comparative Example 4 and Example 4 using the purified lanolin as the base material, there was no fishy odor.

For the samples using the hydrophilic ointment as the base material, Comparative Example 5 generated a strong fishy odor after storage at 40°C for 2 weeks, and the odor lasted thereafter. Example 5 also generated a fishy odor, but the odor was weaker than that of Comparative Example 5 stored under the same conditions.

For the samples using the urea cream as the base material, Comparative Example 6 generated a strong fishy odor after storage at 40°C for after 2 weeks, and the odor became stronger with time until 3 months had elapsed. In contrast, Example 6 was able to substantially completely prevent the generation of fishy odor even when it had been stored at 40°C for 3 months.

In Comparative Example 7 and Example 7 using the water-adsorbable ointment as the base material, there was no fishy odor.

It was confirmed that the antioxidant preparation of the present invention can prevent the generation of fishy odor in the white petrolatum, white ointment, zinc oxide ointment, hydrophilic ointment, and urea cream. In the purified lanolin and the water-adsorbable ointment, there were no differences in the sensory test. This is because these base materials themselves have strong odors, and these odors masked fishy odor.

Specifically, when the antioxidant of the present invention was used in any of the base materials except the hydrophilic ointment, odors resulting from oxidation did not generate in terms of sensory testing, even though the samples were stored at 40°C for 3 months. The storage at 40°C for 3 months corresponds to storage at room temperature for one year. Even in the hydrophilic ointment, the results were better than those of the corresponding comparative example.

### [Oxidation Analysis Method]

Oxidation of each external composition was evaluated by measuring the peroxide value (PV) and the quantities of malondialdehyde and alkenal with a kit Saftest^{R} (produced by Saftest Inc. in the U.S., kit for performing a sequence from extraction to oxidation analysis of lipid).

To 1 g of a sample ointment was added 3 mL of Preparation Reagent (Saftet Inc.). The mixture was heated at 55°C for 5 minutes, then strongly shaken, and further heated at 55°C for 15 minutes. Then, the mixture was centrifuged (3000 rpm, 5 minutes), and the supernatant was filtrated through Membrane Sparation Unit (Saftest Inc.). The resulting filtrate was used as the sample solution.
(1) PV measurement
   Into a test tube was taken 50 µL of the prepared sample solution, and Peroxysafe Reagents A, B, and C (Saftet Inc.) were further added. After the mixture was allowed to stand at room temperature (18 to 25°C) for 15 minutes, the absorbance of the mixture was measured to obtain the PV with Saftest™ Analyzer (Saftest Inc.).
(2) Measurement of Malondialdehyde Quantity
   Into a test tube was taken 150 µL of the prepared sample solution, and Aldesafe Reagents A and B (Saftet Inc.) were further added. After the mixture was allowed to stand at 40°C for 90 minutes, the absorbance of the mixture was measured to obtain the quantity of malondialdehyde with Saftest™ Analyzer (Saftest Inc.).
(3) Measurement of Alkenal Quantity

Into a test tube was taken 200 µL of the prepared sample solution, and Alkalsafe Reagenta A and B (Saftet Inc.) were further added. After the mixture was allowed to stand at room temperature (18 to 25°C) for 20 minutes, the absorbance of the mixture was measured to obtain the quantity of alkenal with Saftest™ Analyzer (Saftest Inc.).

### Results

Tables 12, 13, and 14 show the measurement results for PV, malondialdehyde, and alkenal, respectively. Since the background values are different depending on the base material used, the results are shown by differences in value between the examples and the comparative examples after storage for 2 months. For PV (Table 12), the; examples more suppressed the increase in PV value than the comparative examples, except for the case of the urea cream. For malondialdehyde (Table 13), the examples more suppressed the generation of malondialdehyde than the comparative examples, in all the base materials. For alkenal (Table 14), the examples more suppressed the generation of alkenal than the comparative examples, in all the base materials. These results show, chemically as well as in sensory test, that the antioxidant of the present invention has effective antioxidant properties.

As for the purified lanolin and the water-adsorbable ointment, since their background values were high, appropriate measurements were impossible.

**Table 12:**

| PV (meq/kg) | |
|---|---|
| | Difference (Comparative Example - Example) |
| White petrolatum | 0.3 |
| White ointment | 0.8 |
| Zinc oxide ointment | 0.7 |
| Hydrophilic ointment | 0.2 |
| Urea cream | 0.0 |

**Table 13:**

| malondialdehyde (nmol/mL) | |
|---|---|
| | Difference (Comparative Example - Example) |
| White petrolatum | 7.5 |
| White ointment | 6.0 |
| Zinc oxide ointment | 2.9 |
| Hydrophilic ointment | 2.2 |
| Urea cream | 1.3 |

**Table 14:**

| alkenal (nmol/mL) | |
|---|---|
| | Difference (Comparative Example - Example) |
| White petrolatum | 27.6 |
| White ointment | 22.2 |
| Zinc oxide ointment | 21.1 |
| Hydrophilic ointment | 7.9 |
| Urea cream | 7.3 |

### Experimental Example

In addition to the combined use of sesamol and ascorbyl palmitate, exhibiting effective properties in the above examples, other formulas providing similar properties will now be described in the following experimental examples.

### Experimental Example 1

### <Test for effect of sesamol + ascorbyl palmitate + δ-tocopherol>

The following antioxidant preparations were added separately to refined fish oil (containing 0.5% by weight of δ-tocopherol) to prepare samples.
· sesamol (1.0% by weight) + ascorbyl palmitate (0.01% by weight)
· sesamol (1.0% by weight) alone
· ascorbyl palmitate (0.01% by weight) alone

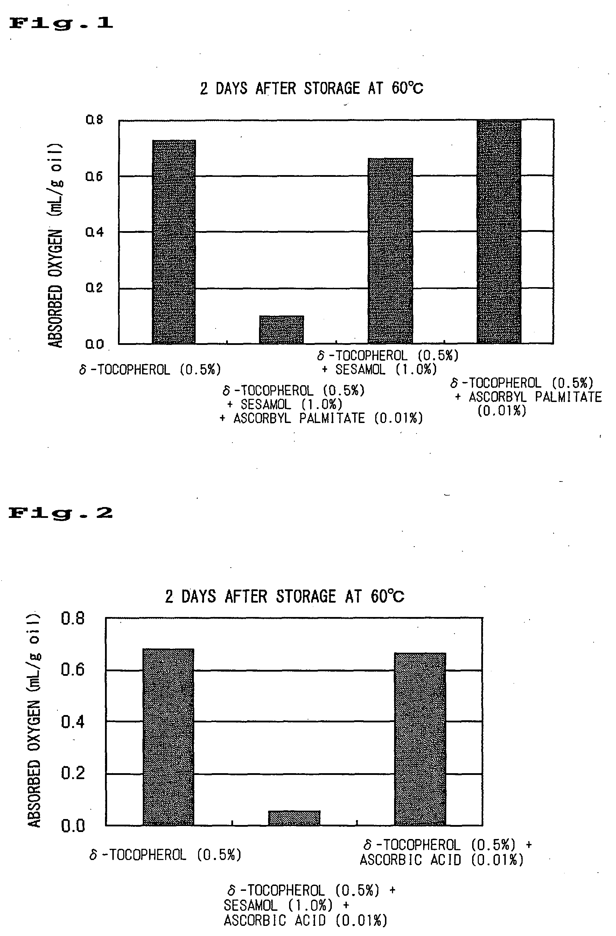

After 3 mL of the samples were placed separately in respective 30 mL brown bottles and hermetically sealed with septums, the samples were stored at 60°C. After 2 days, the concentration of oxygen in the headspace was measured by gas chromatography and the amount of oxygen absorbed by (reacted with) the oil was calculated. The results are shown in Fig. 1. Fig. 1 shows that the combined use of sesamol and ascorbyl palmitate in the refined fish oil reduced the amount of absorbed oxygen and much more increased the oxidative stability of the refined fish oil than the case where sesamol or ascorbyl palmitate was singly added.

### Experimental Example 2

### <Test for effect of sesamol + ascorbic acid + δ-tocopherol>

The following antioxidant preparations were added separately to refined fish oil (containing 0.5% by weight of δ-tocopherol) to prepare samples.
· sesamol (1.0% by weight) + ascorbic acid (0.01% by weight)
· ascorbic acid (0.01% by weight) alone

After 4 mL of the samples were placed separately in respective 30 mL brown bottles, storage tests were performed in the same manner as in Experimental Example 1. The results are shown in Fig. 2. Fig. 2 shows that the combined use of sesamol and ascorbic acid in the refined fish oil reduced the amount of absorbed oxygen and much more increased the oxidative stability of the refined fish oil than the case where ascorbic acid was singly added.

### Experimental Example 3

### <Content Dependence of Sesamol and Ascorbyl Palmitate>

The antioxidant preparations constituted of δ-tocopherol, sesamol, and ascorbyl palmitate in the following proportions were added separately to refined fish oil (containing 0.5% by weight of δ-tocopherol) to prepare samples.
· 0.5%:0.5%:0.05%
· 0.5%:0.5%:0.1%
· 0.5%:1.0%:0.05%
· 0.5%:1.0%:0.1%

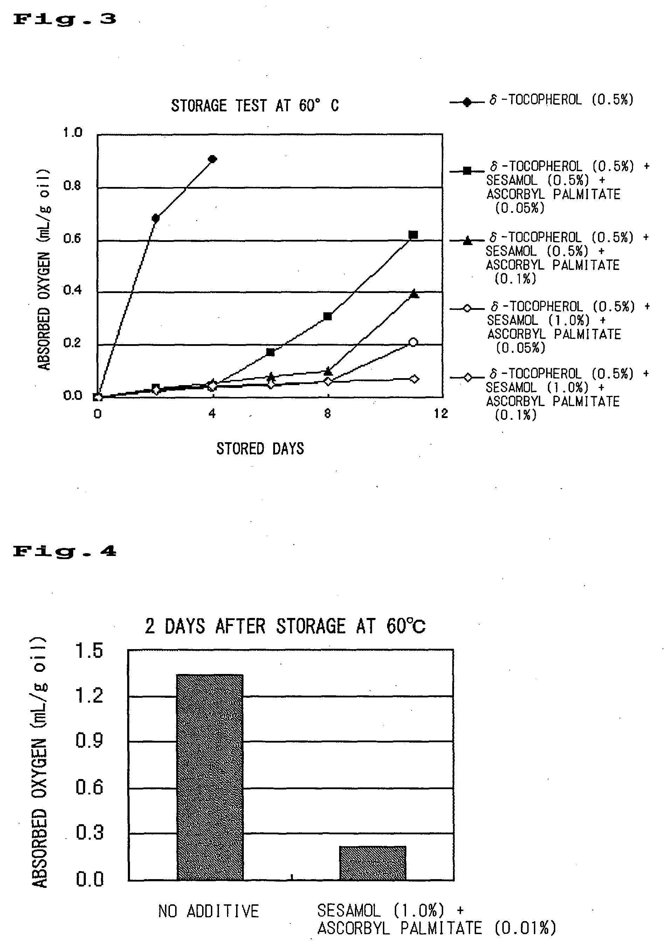

The samples were subjected to the same storage test as in Experimental Example 2 for 11 days. Fig. 3 shows the results. The results suggest that as the contents of sesamol and ascorbyl palmitate in the refined fish oil are increased, the amount of absorbed oxygen is reduced, and that the oxidative stability of the refined fish oil can be increased with content dependence.

### Experimental Example 4

### <Test for Effect of sesamol + ascorbyl palmitate>

An antioxidant preparation of sesamol (1.0% by weight) + ascorbyl palmitate (0.01% by weight) was added to refined fish oil (not containing δ-tocopherol) to prepare a sample. The resulting sample was subjected to the same storage test as in Experimental Example 1. The results are shown in Fig. 4. It has been found that the combined used of sesamol and ascorbyl palmitate can reduce the absorption of oxygen and greatly increase the oxidative stability of the refined fish oil, without using tocopherol.

### Experimental Example 5

### <Test for Effect of Sesame Oil Extract + Ascorbyl Palmitate (0.1% by weight) + δ-tocopherol>

To 8.23 g of roasted sesame oil was added 100 mL of methanol, and the mixture was strongly agitated. Then, methanol was evaporated from the methanol phase to obtain 0.28 g of extract. It was confirmed that this extract from methanol contained sesamol, by thin layer chromatography (thin layer: Kiesolgel 60 F254, 0.25 mm, produced by Merck & Co., Inc.; developing solvent: hexane:diethyl ether:acetic acid = 70:30:1; coloring reagent: 1,1-diphenyl-2-picrylhydrazyl, free Radical).

The following antioxidant preparations were added separately to refined fish oil (containing 0.5% by weight of δ-tocopherol) to prepare samples.
· methanol extract of sesame oil (2.0% by weight) + ascorbyl palmitate (0.1% by weight)
· methanol extract of sesame oil (2.0% by weight) alone
· ascorbyl palmitate (0.1% by weight) alone

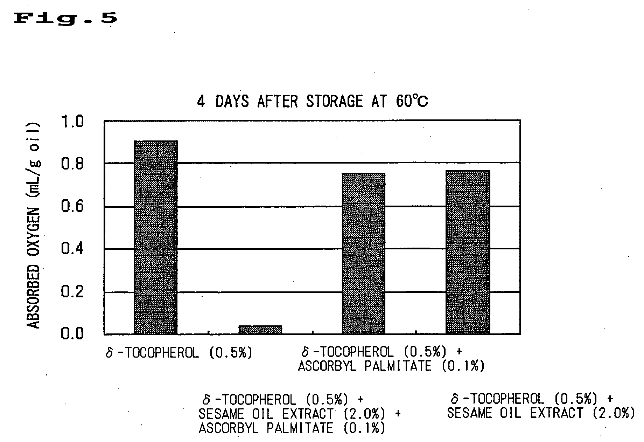

The samples were subjected to the same storage test as in Experimental Example 2. The results are shown in Fig. 5. It has been found that the methanol extract of sesame oil as well as sesamol does not produce the effect by itself, and that combined use with ascorbyl palmitate reduces the amount of absorbed oxygen and increases the oxidative stability of the refined fish oil.

### Experimental Example 6

### <Test for Effect of Roasted Sesame residue Extract + Ascorbyl Palmitate + δ-tocopherol>

### (1) Roasted sesame residue extract 1

To 1.0 kg of roasted sesame residue was added 2.0 kg of 95% ethanol, and the mixture was strongly agitated at 40°C for 2 hours. Then, the roasted sesame residue was filtrated to obtain an extract. To the roasted sesame residue subjected to the filtration, 1.5 kg of 95% ethanol was added again. The mixture was strongly shaken at 40°C for one hour, and then filtrated to obtain an extract. The total extract obtained by 2 cycles of filtration was concentrated, and 240 g of ethyl acetate and 80 g of water were added, followed by strongly shaking at 45°C for 1 hour. After shaking, the water phase was removed, and 40 g of propylene glycol monooleate was added to the ethyl acetate phase. The ethyl acetate was evaporated to yield 58 g of roasted sesame residue extract 1 (18 g of roasted sesame residue extract in real terms because the extract contained 40 g of propylene glycol monooleate).

### (2) Roasted Sesame residue Extract 2

To 1.0 kg of roasted sesame residue was added 2.0 kg of 95% ethanol, and the mixture was strongly agitated at 40°C for 2 hours. Then, the roasted sesame residue was filtrated to obtain an extract. To the roasted sesame residue subjected to the filtration, 1.5 kg of 95% ethanol was added again. The mixture was strongly shaken at 40°C for one hour, and then filtrated to obtain an extract. To the total extract obtained by 2 cycles of filtration was added 40 g of propylene glycol monooleate. Then, the 95% ethanol was evaporated to yield 50 g of roasted sesame residue extract (10 g of roasted sesame residue extract in real terms because the extract contained 40 g of propylene glycol monooleate).

### (3) Roasted Sesame residue Extract 3

To 200 g of roasted sesame residue was added 300 mL of 95% ethanol, and the mixture was strongly shaken at 40°C for 2 hours. Then, the roasted sesame residue was filtrated to obtain an extract. To the roasted sesame residue subjected to the filtration, 300 mL of 95% ethanol was added again. Then, the mixture was strongly agitated at 40°C for 2 hours, and then filtrated to obtain an extract. The total extract obtained by 2 cycles of filtration was concentrated. Then, 150 mL of ethyl acetate and 50 mL of water were added, and the mixture was strongly agitated at room temperature for one hour. After the agitation, the water phase was removed, and further ethyl acetate was evaporated to yield 9.0 g of roasted sesame residue extract.

Roasted sesame residue extract 1 was subjected to a measurement by high-performance liquid chromatography with an electrochemical detector. The measurement was performed under the following conditions. The chart was shown in Fig. 6. Since the peaks were detected by the electrochemical detector, all the substances represented by the peaks have antioxidant properties. Thus, it is shown that the roasted sesame residue contains many antioxidative components, including sesamol and pinoresinol.
Measurement conditions
Column: TSK-gel ODS-80Ts 4.6 × 150 mm
Eluant:
   0-5 min., methanol:water (containing 2% of 1 M ammonium acetate buffer (pH 4.4)) = 40:60
   10-17 min., methanol:water (containing 2% of 1 M ammonium acetate buffer (pH 4.4)) = 70:30
   22-40 min., methanol:water (containing 2% of 1 M ammonium acetate buffer (pH 4.4)) = 100:0
Flow rate: 1.0 mlL/min.
Column temperature: 35°C
Sample concentration: 10-12 mg/mL
Sample solvent: methanol:ethanol:hexane = 5:4:1 Injection volume: 10 µL
Electrode 1 (reduction potential): -1 V
Electrode 2 (oxidation potential): 500 mV
Range: 20 µA

Samples were prepared by adding the following antioxidant preparations separately to refined fish oil (containing 0.5% by weight of δ-tocopherol).
· roasted sesame residue extract 1 (1.0% by weight) + ascorbyl palmitate (0.05% by weight)
· roasted sesame residue extract 1 (1.0% by weight) + ascorbyl palmitate (0.1% by weight)
· roasted sesame residue extract 1 (1.0% by weight) alone · ascorbyl palmitate (0.1% by weight) alone
· roasted sesame residue extract 2 (1.0% by weight) + ascorbyl palmitate (0.1% by weight)
· roasted sesame residue extract 3 (1.0% by weight) + ascorbyl palmitate (0.1% by weight)

The samples were subjected to the same storage test as in Experimental Example 2. The results are shown in Fig. 7. It has been found that the roasted sesame residue extracts can reduce the amount of absorbed oxygen and increase the oxidative stability of the refined fish oil, by using them in combination with ascorbyl palmitate, as in the case of sesamol.

### Experimental Example 7

### <Test for Effect of Roasted Sesame residue Extract + Ascorbyl Palmitate + δ-tocopherol>

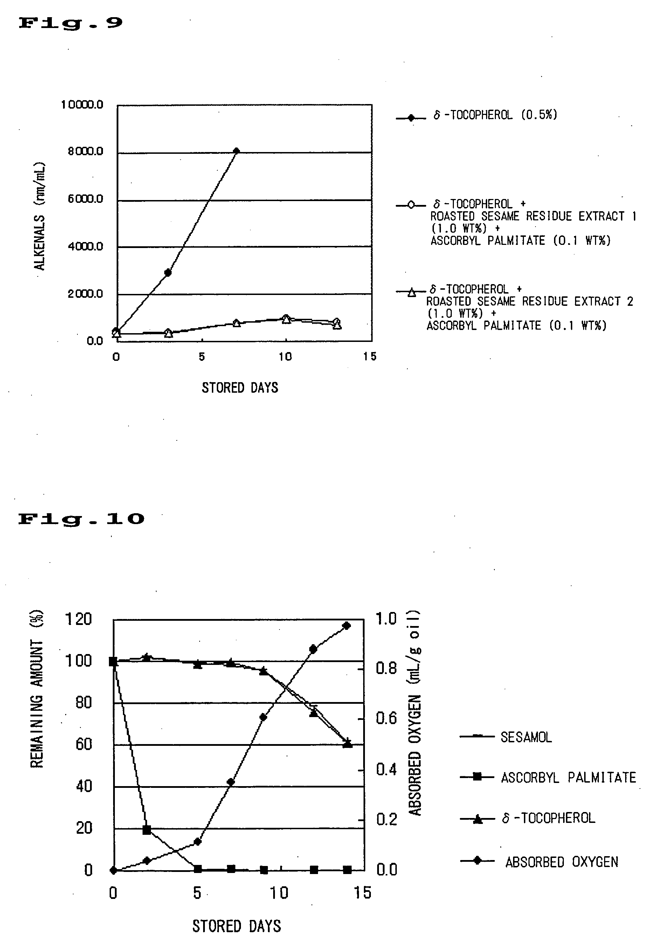

Samples were prepared by separately adding antioxidant preparations of Experimental Example 6: roasted sesame residue extract 1 (1.0% by weight) + ascorbyl palmitate (0.1% by weight); and roasted sesame residue extract 2 (1.0% by weight) + ascorbyl palmitate (0.1% by weight). In 20 mL brown bottles were placed 10 mL of the samples separately. Each mixture was stored at 60°C with the bottle open, and changes in concentration of malondialdehyde and alkenals were measured during storage with SafTest produced by Saftest Inc. The results are shown in Figs. 8 and 9. These figures show that by adding a roasted sesame residue extract and ascorbyl palmitate to refined fish oil, the generation of malondialdehyde and alkenals resulting from oxidation decomposition of fish oil, which are odorants of deteriorated fish oil, can be highly suppressed and thus the fish oil can be stabilized.

### Experimental Example 8

Sesaminol obtained from roasted sesame residue extract 3 with a silica gel open column and ODS-HPLC was used instead of the sesamol of Experimental Example 1. As a result, the same effect was produced.

### Experimental Example 9

Pinoresinol obtained from roasted sesame residue extract 3 with a silica gel open column and ODS-HPLC was used instead of the sesamol of Experimental Example 1. As a result, the same effect was produced.

### Experimental Example 10

2,3-Di(4'-hydroxy-3'-methoxybenzyl)-2-buten-4-olide obtained from roasted sesame residue extract 3 with a silica gel open column ODS-HPLC was used instead of the sesamol of Experimental Example 1. As a result, the same effect was produced.

### Experimental Example 11

### <Test for Effect of Excessive Amount of Ascorbic Acid Ester>

The following antioxidant preparations were added separately to refined fish oil (containing 0.5% by weight of δ-tocopherol) to prepare samples.
· sesamol (0.5%) + ascorbyl palmitate (0.1%)
· sesamol (1.0%) + ascorbyl palmitate (0.1%)

After 4 mL of the samples were placed separately in respective 30 mL brown bottles and hermetically sealed with septums, the samples were stored at 60°C. The concentration of oxygen during storage was measured by gas chromatography, and thus the amount of oxygen reacted with (absorbed by) the oil was calculated. Also, the remaining antioxidant contents were analyzed by HPLC with an electrochemical detector.

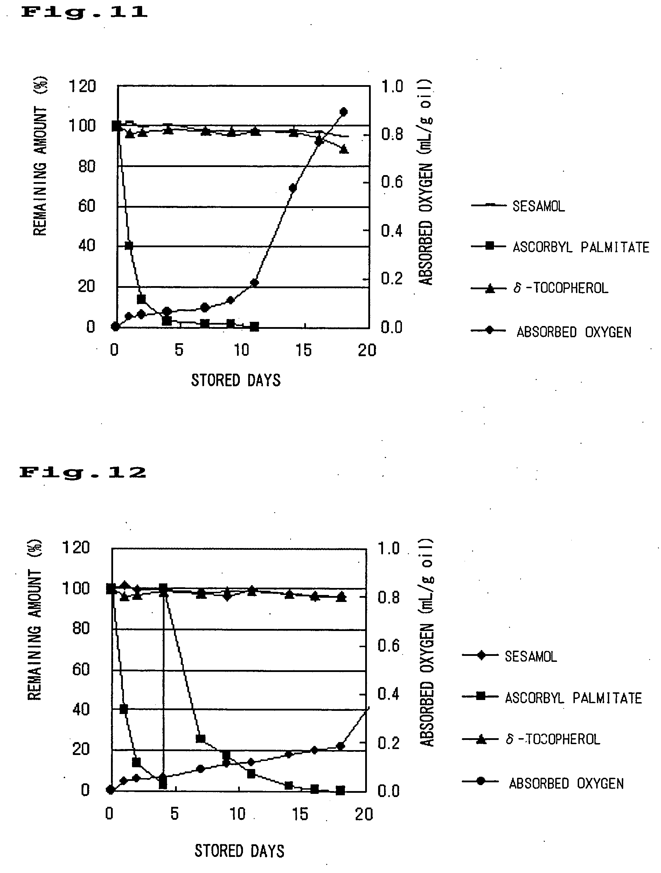

The results are shown in Figs. 10 and 11. The figures show that, in either case, ascorbyl palmitate was consumed (oxidized) to be lost first, and then the refined fish oil, δ-tocopherol, and sesamol simultaneously started oxidizing. It is therefore assumed that ascorbyl palmitate plays an important role when the antioxidant preparation contains δ-tocopherol, sesamol, and ascorbyl palmitate. In order to confirm this assumption, 0.1% of ascorbyl palmitate was further added to the same system as in Fig. 11 on day four. The results are shown in Fig. 12. Fig. 11 shows that the oil was not oxidized until ascorbyl palmitate was consumed (around day ten); Fig. 12, in which ascorbyl palmitate was added on day four, shows that oxidation of the oil was continuously suppressed even after day ten.

Thus, it has been shown that it is important that ascorbyl palmitate is present in the antioxidant system of the present invention.

### Experimental Example 12

### <Tests for Effect on Eicosapentaenoic Acid Ethyl Ester of the Present Invention, and for Effect of Excessive Amount of Ascorbyl Palmitate>

Samples were prepared by adding the following antioxidant preparations separately to eicosapentaenoic acid ethyl ester with a purity of 99% (containing 0.2% of α-tocopherol).
· sesamol (1.0%) + ascorbyl palmitate (0.1%)
· sesamol (1.0%) + ascorbyl palmitate (0.5%)

Each sample was stored at 60°C, and the peroxide value (PV) was measured.

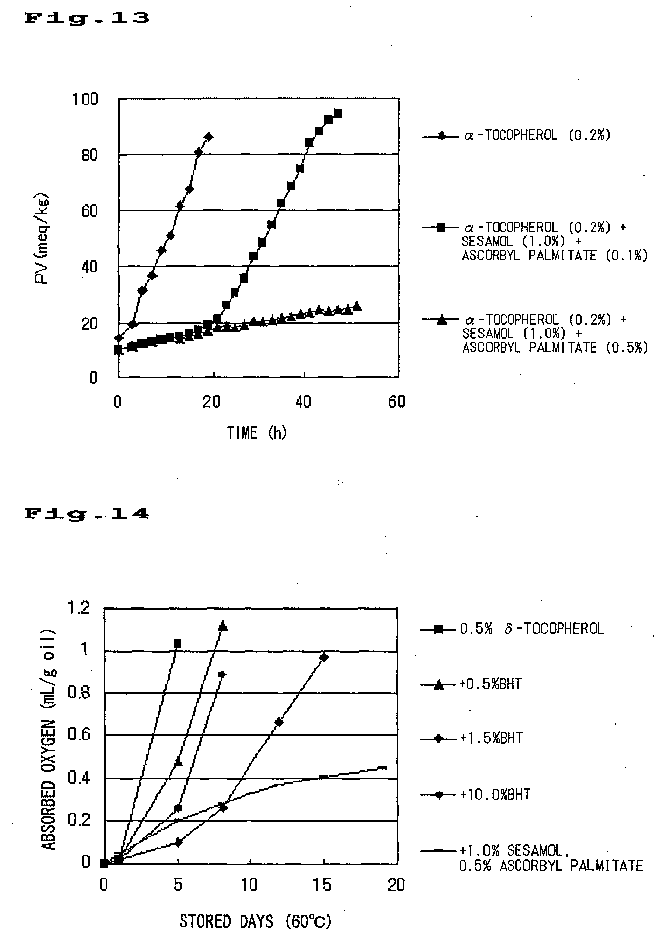

The results are shown in Fig. 13. The antioxidant property of the antioxidant preparation containing an excessive amount of ascorbyl palmitate (0.5%) was maintained in comparison with that of the antioxidant preparation containing a soluble amount of ascorbyl palmitate (0.1%).

### Experimental Example 13

### <Comparison with antioxidant (t-butylhydroxytoluene (BHT)) generally used in external preparations)>

### (1) Comparison of Effects in Refined Bulk Oil

The antioxidant preparation of the present invention was compared with t-butylhydroxytoluene (BHT), which is an antioxidant generally used in external preparations. Samples were prepared by adding 0.5%, 1.5%, or 10.0% of BHT, or 1.0% of sesamol and 0.5% of ascorbyl palmitate to refined fish oil (sardine oil) containing 0.5% of δ-tocopherol. To 30 mL brown bottles, 4 mL of the samples were placed separately. The bottles were hermetically sealed with septums and stored at 37°C. The oxygen concentration in the headspace was measured with time by gas chromatography and thus the amount of oxygen reacted with (absorbed by) the oil was calculated.

### Results

Fig. 14 shows the comparison of the antioxidant properties in the bulk oil between BHT and the antioxidant preparation of the present invention. The results show that the antioxidant preparation of the present invention exhibited higher antioxidant property than the same amount (1.5%) of BHT as the total amounts of the antioxidants in the antioxidant preparation of the present invention, and also than a much larger amount (10.0%) of BHT.

### (2) Comparison of effects in External Preparation

To refined fish oil were added 0.5%, 1.5%, and 10.0% of BHT separately. External preparations were prepared by adding the BHT-containing fish oils samples separately to a white petrolatum acting as the base material, in the same manner as in Example 1. The resulting preparations were subjected to storage test at 40°C, and evaluated by the same sensory test (n=3) as above.

### Results

Each of the samples using 0.5%, 1.5%, and 10.0% of BHT generated a fishy odor in storage for 2 weeks. As shown in Table 15, the sample using antioxidant preparation of the present invention did not generate any fishy odor even in storage for 1 month. Hence, the antioxidant preparation of the present invention can more produce the effect of preventing fishy odor than BHT.

**Table 15**

| | 0.5% BHT | 1.5% BHT | 10.0% BHT | Example 1 |
|---|---|---|---|---|
| After 1 week | 0.7±0.9 | 0.3±0.5 | 1.0±0.8 | |
| After 2 weeks | 2.3±0.9 | 2.3±0.5 | 1.3±0.5 | 0.0±0.0 |
| After 3 weeks | 4.0±0.0 | 3.3±0.5 | 2.7±0.5 | |
| After 4 weeks | | | | 0.3±0.5 |

### INDUSTRIAL APPLICABILITY

The present invention can provide an external composition having oxidative stability in spite of containing a polyunsaturated fatty acid or its salt or ester. The present invention can also provide an external medical drug, an external quasi drug, and a cosmetic preparation that contain a polyunsaturated fatty acid or the like, such as EPA, the use of which has been conventionally limited due to an odor resulting from oxidation.

## Claims

1. An external composition having oxidative stability comprising: a polyunsaturated fatty acid or its salt or ester which contains an antioxidant comprising an antioxidative sesame component and ascorbic acid or an ascorbyl fatty acid ester; and an external base material.

2. The external composition according to Claim 1, wherein the antioxidative sesame component is at least one of the substances represented by peaks detected by high-performance liquid chromatography using an electrochemical detector at elution times of about 2.66, 3.40, 3.84, 4.57, 4.98, 5.82, 7.00, 8.67, 9.84, 11.24, 12.29, 12.49, 13.36, 14.04, 14.32, 14.74, 15.22, 15.60, 15.82, 16.34, 16.98, 18.10, 18.43, and 34.91 minutes.

3. The external composition according to Claim 1, wherein the antioxidative sesame component is extracted from sesame, sesame oil, or sesame residue, using a solvent, a lipid, or an emulsifier singly or in combination.

4. The external composition according to Claim 1, wherein the antioxidative sesame component is at least one selected from the group consisting of sesamol, sesaminol, episesaminol, pinoresinol, epihinoresinol, syringaresinol, samine, sesamolinol, and 2,3-di(4'-hydroxy-3'-methoxybenzyl)-2-buten-4-olide.

5. The external composition according to any one of Claims 1 to 4, wherein the ascorbyl fatty acid ester contains ascorbyl palmitate or ascorbyl stearate.

6. The external composition according to any one of Claims 1 to 5, wherein the ascorbic acid or the ascorbyl fatty acid ester is contained in an excessive amount more than the amount soluble in the polyunsaturated fatty acid or its salt or ester.

7. The external composition according to Claim 6, wherein the excessive amount of the ascorbic acid or the ascorbyl fatty acid ester is in a powder or solid form.

8. The external composition according to any on of Claims 1 to 7, wherein the polyunsaturated fatty acid contains at least one of eicosapentaenoic acid and docosahexaenoic acid.

9. The external composition according to any one of Claims 1 to 8, wherein the ester of the polyunsaturated fatty acid is a triglyceride containing the polyunsaturated fatty acid as a constituent, or a lower alcohol ester of the polyunsaturated fatty acid.

10. The external composition according to any one of Claims 1 to 8, wherein the ester of the polyunsaturated fatty acid is added in a form of refined fish oil.

11. The external composition according to any one of Claims 1 to 10, further comprising tocopherol.

12. The external composition according to any one of Claims 1 to 11, wherein the external composition is used for a skin inflammatory disease.

13. The external composition according to any one of Claims 1 to 12, wherein the external base material is an oleaginous base material such as white ointment, white petrolatum, or zinc oxide ointment; or an emulsion base material (cream), such as hydrophilic ointment, purified lanolin, water-adsorbable ointment, or urea cream.

14. The external composition according to Claim 13, wherein the external base material is a white ointment, a white petrolatum, a urea cream, or a zinc oxide ointment.

15. The method for producing an external composition having oxidative stability, containing a polyunsaturated fatty acid or its salt or ester, wherein an antioxidative sesame component, ascorbic acid or an ascorbyl fatty acid ester, and an external base material are mixed with the polyunsaturated fatty acid or its salt or ester, or an oil or fat containing the polyunsaturated fatty acid or its salt or ester, under conditions preventing oxygen from mixing, and the mixture is quickly placed in a hermetic container, and wherein the polyunsaturated fatty acid or its salt or ester has an peroxide value (PV) of 3.0 meq/kg or less and an acid value (AV) of 1.0 or less, and no odor in terms of sensory testing.

16. The method according to Claim 15, wherein the conditions preventing oxygen from mixing is an atmosphere purged with an inert gas.
